# EUROPEAN PATENT APPLICATION

(11) **EP 2 823 753 A1**
(43) Date of publication of application: **14.01.2015**
(21) Application number: 13176360.9
(22) Date of filing: 12.07.2013
(51) Int. Cl.: A61B 5/00, A61B 5/145

(54) **Graphical user interface for blood glucose analysis**

(71) Applicant: Axon Limited, Southampton Hampshire SO15 2AT (GB)
(72) Inventor: Stott, Jonathan, Sittingbourne, Kent ME9 7AZ (GB)
(74) Representative: Keseris, Denis

(57) **Abstract**

A graphical user interface for displaying blood glucose information is disclosed. The graphical user interface comprises a trend window arranged to display blood glucose levels of a user on a timeline and a magnifying window superimposed on a segment of the timeline, the relative position of the magnifying window with respect to the timeline being movable. The graphical user interface also comprises a magnified window arranged to display, over an area which is larger than the magnifying window, the information contained in the segment of the timeline and at least one contextual information window arranged to display, over an area of the same size as the magnified window, information which is contextual to the blood glucose levels.

## Description

### Technical Field

The present invention relates to the field of graphical user interfaces, and more particularly to the field of graphical user interfaces for use in analysing glucose levels in the blood.

### Background to the Invention and Prior Art

The increasing prevalence of diagnosed diabetes in countries around the world has lead to an need to empower diabetics and their healthcare professionals with the ability to quickly and accurately analyse blood glucose levels collected over a period of time. Moreover, the promotion of preventative medicine has led to the desire of healthcare professionals and diabetes suffers to understand how various lifestyle choices can affect levels of glucose in the blood. Furthermore, with recent advancements in mobile data-communications, new systems of blood glucose monitoring can now be run on powerful mobile devices such as mobile telephones, smartphones, PDAs and tablet computers.

Known blood glucose monitoring systems provide means for graphically representing blood glucose levels over time. For example, United States patent application publication US2010095229A1 discloses a graphical user interface which shows selectable blood glucose readings over time. The time period over which the glucose readings can be shown is selectable by a user. For example, a number of selectable readings can be shown over a period of either 1 day, 7 days or 21 days. When a reading is selected by the user, the graphical user interface shows a bubble which provides contextual information about the specific glucose reading that is selected. This contextual information can include an indication as to whether the reading was taken before or after a meal, or the dosages of insulting taken. Similar graphs can be provided for insulin dosage versus time, or versus glucose levels.

Prior art graphical user interfaces can thus provide a user with the ability to determine blood glucose trends by viewing a large number of readings over an extended period of time. Moreover, prior art interfaces can also provide users with detailed contextual information of individual blood glucose readings, thereby allowing users to take into account causal information in relation to individual readings.

Because these systems show contextual information for each reading on an individual basis however, and because blood glucose trends can only be determined by observing a large number of data points, a significant problem with prior art interfaces is the trade-off between a user's ability to identify trends by viewing a large number of readings over an extended period of time, and a user's ability to quickly analyse contextual information.

This inherent trade-off in prior interfaces creates a problem because, in order to identify a trend, a user must be able to see a large number of individual readings. The amount of contextual information in respect of this large number of individual readings that can then be viewed however is limited by the physical size of the screen on which the information is being viewed. This limitation of the physical screen size, coupled with the user's need to quickly switch between large scale overview information and details contextual information, puts an undue cognitive burden on the user.

### Summary of the Invention

The present invention solves the problems associated with the prior art, by providing a graphical user interface for displaying blood glucose information, the graphical user interface comprises: a trend window arranged to display blood glucose levels of a user on a timeline; a magnifying window superimposed on a segment of the timeline, the relative position of the magnifying window with respect to the timeline being movable; a magnified window arranged to display, over an area which is larger than the magnifying window, the information contained in the segment of the timeline; and at least one contextual information window arranged to display, over an area of substantially the same size as the magnified window, information which is contextual to the blood glucose levels.

The graphical user interface preferably further comprises: resizing means arranged to allow a user to resize the magnifying window with respect to the trend window, wherein the resizing of the magnifying window with respect to the trend window changes the size of the segment of the timeline.

The graphical user interface is preferably arranged such that a user can reposition the timeline within the trend window.

The graphical user interface preferably further comprises: magnifying window moving means arranged to move the magnifying window with respect to the trend window, the relative position of the magnifying window with respect to the timeline being moved by the magnifying window moving means.

The graphical user interface preferably further comprises: a plurality of contextual information windows arranged to display, over an area of the same size as the magnified window, information which is contextual to the blood glucose levels, the plurality of contextual information windows being arranged contiguously and in a particular order on the graphical user interface.

Preferably, each of the plurality of contextual information windows is arranged to be moveable, such that the particular order in which the contextual information windows are arranged can be changed by a user.

The graphical user interface preferably further comprises at least one contextual information window including a graphical input icon which is arranged to be dragged and dropped by a user onto a location of the at least one contextual information window, thereby creating an added reading to the at least one contextual information window, the location of the at least one contextual information window being indicative of quantum and time of the added reading being input by the user into the at least one contextual information window.

Preferably, dragging and dropping of the graphical input icon onto the location of the at least one contextual information window causes a popup window to appear, the popup window being arranged to allow a user to input further information in respect of the added reading and/or to adjust the location of the added reading in the at least one contextual information window.

Preferably, the at least one contextual information window is arranged to display quantitative and qualitative information on a timeline, the qualitative and quantitative information relating to one of: insulin injected by a user, insulin administered to a patient by way of an insulin pump, food consumed by a user and exercise sessions performed by a user.

The present invention further provides an apparatus which comprises: processing means; and a display device, the processing means being arranged to cause the display device to display the aforementioned graphical user interface.

The present invention also provides a method of providing a graphical user interface for displaying blood glucose information, the method comprises: providing a trend window arranged to display blood glucose levels of a user on a timeline; providing a magnifying window superimposed on a segment of the timeline, the relative position of the magnifying window with respect to the timeline being movable; providing a magnified window arranged to display, over an area which is larger than the magnifying window, the information contained in the segment of the timeline; and providing at least one contextual information window arranged to display, over an area of the same size as the magnified window, information which is contextual to the blood glucose levels.

The present invention also provides a computer program product for a data-processing device, the computer program product comprising a set of instructions which, when loaded into the data-processing device, causes the device to perform the steps of the aforementioned method.

As will be appreciated by the skilled reader, the present invention provides several advantages over the prior art. For example, the present invention allows a user to quickly identify contextual information relating to individual blood glucose readings while at the same time situating those individual readings within the context of a broader trend.

Using prior art graphical user interfaces, providing this information would require a very large screen which would contain an enormous amount of information, thereby putting an undue cognitive burden on the user.

Moreover, prior art blood glucose monitoring interfaces show a number of parameters (such as insulin, carbohydrate intake, exercise, and other notes) all on a single display, which make reading and identifying trends in the data very burdensome for the user. The present invention however solves this problem by separating parameters on individual contextual information windows that are aligned, and maintain alignment as the user moves the magnifying window.

Furthermore, the positions of these contextual information windows can be moved with respect to each other, thereby easing the cognitive burden on a user in trying to identifying correlation between readings from different windows.

### Description of the Drawings

Some embodiments of the present invention are now described, by way of example only, and with reference to the accompanying drawings, in which:
FIGURE 1 is a schematic diagram of a data processing system suitable for use with the present invention; and
FIGURE 2 is a representation of a graphical user interface in accordance with one embodiment of the present invention.

### Detailed Description of Embodiments

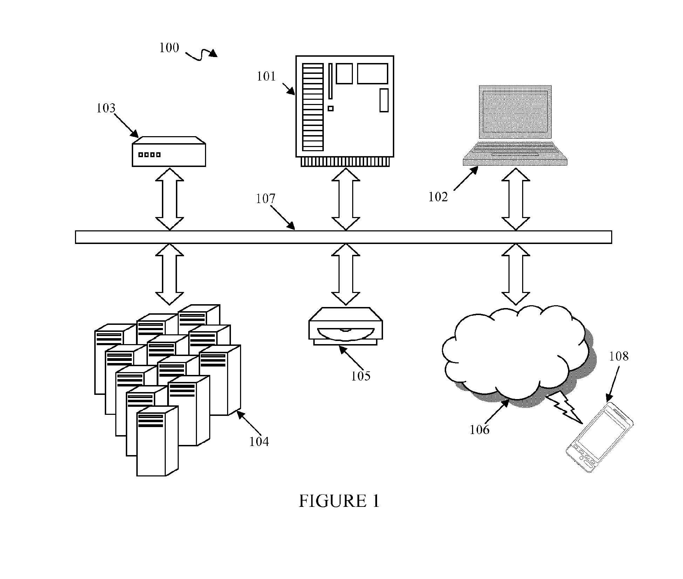

Figure 1 is a schematic diagram of a data processing system 100 suitable for implementing an embodiment of the present invention. The data processing system 100 comprises a processing unit 101, such as a central processing unit (CPU), an input/output device 102, such as a terminal including a screen and a keyboard and a local memory unit 103, such as hard drive. As will be appreciated, in some embodiments, the processing unit 101, the input/output device 102 and the local memory unit 103 can all be incorporated into a single multipurpose desktop or laptop computer.

In some embodiments, the data processing system 100 can also comprise a communication channel 107 for ensuring data communication between elements of the data processing system 100. It will be appreciated that the communication channel 107 can be provided by a local communication channel, such as a Universal Serial Bus (USB), by a telecommunication channel, such as a Local Area Network (LAN) or a Wide Area Network (WAN), or a combination thereof. In some embodiments, the data processing system 100 also comprises a remote memory device 105 for off-site recording of input data and/or a remote storage facility 104 for the remote storage of input data. Finally, data processing system 100 can also be connected to a computer network 106, such as a Local Area Network (LAN) or the Internet, in order to enable communication with a mobile communication device 108 such as a smartphone, PDA or tablet computer.

In some embodiments, the graphical user interface of the present invention can be displayed on the viewing screen of an input/output device 102 and/or the viewing screen of the mobile communication device 108. Similarly, data relating to blood glucose readings and/or contextual information can also be input via input/output device 102 and/or mobile communication device 108, using known data input device such a keyboard or a touch screen. The input data can then be stored on any of the above mentioned memory means, or in the local memory of the mobile communication device 108.

In some embodiments, the processing required to produce a graphical user interface in accordance with the present invention can be performed by the processing unit 101. In another embodiment, the processing required can be performed on the mobile communication device 108 or any other dedicated or shared processing platform (not shown). In yet another embodiment of the invention, the required processing can be performed on either the processing unit 101, the mobile communication device 108, any other dedicated or shared processing platform, or any combination thereof.

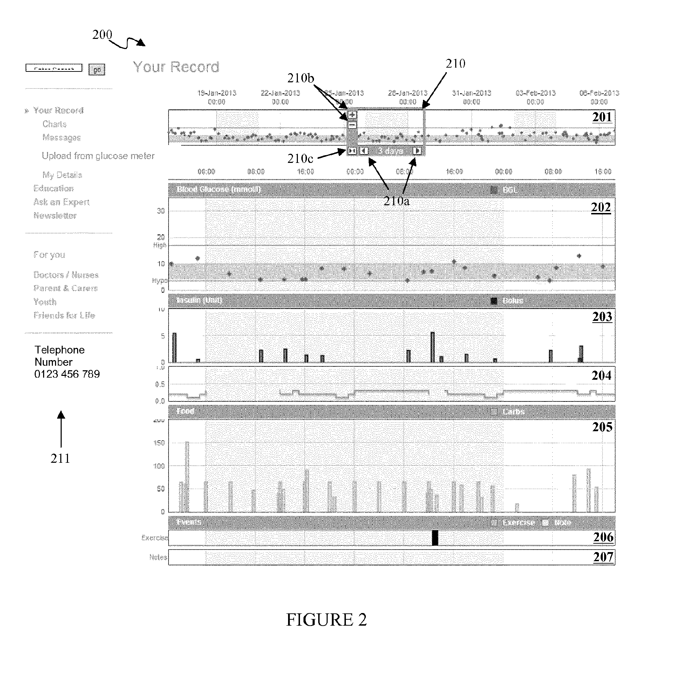

Figure 2 shows a graphical user interface 200 in accordance with one embodiment of the present invention. The graphical user interface 200 includes a trend window 201 that shows a number of blood glucose readings (shown in mmol/l in the embodiment of Figure 2) over a period of time. The blood glucose readings can be input by a user and stored on any of the aforementioned memory means, or in the local memory of the mobile communication device 108.

The trend window 201 of Figure 2 shows a period of 21 days divided into 3-day sections. In the embodiment of Figure 2, both the total length of time shown in the trend window 201, and the number of divisions within that total length of time, are variable by the user. the trend window 201 also shows statistical information in regard to the "High", i.e. hyperglycaemic, and "Hypo", i.e. hypoglycaemic, readings. As will be appreciated by the skilled reader, a number of statistical calculations and trends can be also be shown on the trend window 201, without departing from the scope of the invention.

Overlaid upon trend window 201 is magnifying window 210. Magnifying window 210 is operable to slide along trend window 201 in both directions (i.e. forwards and backwards in time along the timeline). The content of the magnifying window 210 is represented in magnified window 202. The horizontal axis of the magnified window 202 represents the portion of the timeline of trend window 201 captured by the magnifying window 210.

In one embodiment, the magnifying window 210 is operable to move from side to side with respect to the trend window 201. The direction of movement of magnifying window 210 can be controlled by a user by activating the directional control buttons 210a using a cursor controlled by a mouse, or simply by touching either button 210a on a touch screen. Recentering of the magnifying window 210 with respect to the trend window 201 can be achieved by activating recentering button 210c in the same way as directional control buttons 210a.

In another embodiment, the magnifying window 210 is fixed with respect to the trend window 201, and the timeline within trend window 201 can be moved from side to side with respect to both the trend window 201 and the magnifying window 210. In this other embodiment, the direction of movement of the timeline can be controlled by a user by activating the directional control buttons 210a using a cursor controlled by a mouse, or by touching either button 210a on a touch screen. Recentering of the timeline with respect to the trend window 201 and magnifying window 210 can be achieved by activating recentering button 210c in the same way as directional control buttons 210a.

In yet another embodiment, both the timeline and the magnifying window 210 can be moved with respect to the trend window. In this yet other embodiment, the direction of movement of magnifying window 210 can be controlled by a user by activating the directional control buttons 210a using a cursor controlled by a mouse, or by touching either button 210a on a touch screen. Recentering of the magnifying window 210 with respect to the trend window 201 can be achieved by activating recentering button 210c in the same way as directional control buttons 210a. The direction of movement of timeline can be controlled by a user by selecting the timeline using a cursor controlled by a mouse, or by selecting the timeline on a touch screen, and moving it to the left or the right within the trend window 201.

A given size of magnifying window 210 with respect to trend window 201 represents a given proportion of the timeline shown in trend window 201. Thus, the magnifying window captures a portion of the amount of time shown in trend window 201. By activating the window size buttons 210b however, the period of time captured by magnifying window 210 can be changed. Changing the size of the captured time period of the magnifying window 210 will result in a change in the relative size of the magnifying window 210 with respect to the trend window 201.

The portion of the trend window 201 captured by the magnifying window 210 is shown in the magnified window 202. As such, the timescale shown in magnifying window 201 of Figure 2 (i.e. 3 days) represents the total timescale shown in magnified window 202. Because magnifying window 210 will generally be smaller than magnified window 202, the detailed information captured by magnifying window 210 can be viewed in the magnified window 202 over a larger portion of a viewing surface.

Any change in the size or position of magnifying window 210 with respect to trend window 201 is reflected in what is shown in magnified window 202. That is to say that any change in the size or position of magnifying window 210 with respect to trend window 201 results in a change in the number and/or locations of the blood glucose readings captured in the magnifying window 210. Because the content of the magnifying window 210 is also displayed on the magnified window 202, albeit on a larger physical portion of the viewing screen, variations in the number and/or locations of the blood glucose readings in the magnifying window 210 (resulting from changes in the relative position of the magnifying window 210 with respect to the timeline) will also be displayed on the magnified window 202.

Contextual information windows 203, 204, 205, 206 and 207 are disposed below and in line with magnified window 202, and display events on the same timescale as the magnified window 202. Accordingly, any changes to the magnified window 202 timescale result in corresponding changes to the timescales of contextual information windows 203, 204, 205, 206 and 207. Figure 2 shows a number of particularly advantageous contextual information windows, though the skilled reader will appreciate that other types of information can be shown in contextual information windows.

Contextual information window 203 displays the times at which a user may have injected insulin. The horizontal axis of the window represents the time at which insulin is injected and displays the same timescale as magnified window 202. The vertical axis of the display represents the number of units of insulin injected. The amount of insulin, or "bolus", injected and the time at which the insulin was injected can be input by the user and stored on any of the aforementioned memory means, or in the local memory of the mobile communication device 108.

Contextual information window 204 shows an indication of the insulin that is administered to a patient by way of an insulin pump (a device worn with a catheter that drip feeds insulin continuously). This contextual information can also be input by the user and stored on any of the aforementioned memory means, or in the local memory of the mobile communication device 108. Information from an insulin pump can also be retrieved from the insulin pump itself.

Contextual information window 205 displays the times at which a user may have consumed food. The horizontal axis of the window represents the time at which food is consumed and displays the same timescale as magnified window 202. The vertical axis of the display represents the number of calories of food consumed. Alternatively, the vertical axis of the display could represent an indication of the glycaemic index (GI) of food that has been consumed. The calories consumed and the time at which the calories were consumed can be input by the user and stored on any of the aforementioned memory means, or in the local memory of the mobile communication device 108.

An advantageous aspect of this embodiment is that, by selected the squares in the title bars of the contextual information windows (e.g. Bolus, Carbs, Exercise, and Notes), and dragging them onto their respective window, a user will be able to enter a new reading. The location on the window (i.e. vertical and horizontal axis orientation), will determine the initial quantum and time for the reading, though these can also then be readjusted manually (through a popup window or the like). For example, if a user selected the "bolus" box and dragged it to 0:00 on horizontal line 5, this would create a reading indicative of 5 Units at midnight, though a subsequent popup window could preferably allow a user to adjust these figures immediately. As mentioned above however, in some embodiments, insulin figures and other information would typically be provided to the system automatically.

Contextual information window 206 shows the times at which an exercise session was performed by a user. The vertical axis of contextual window 206 represents a quantitative indication the exercise performed, for example, "light", "moderate", "heavy", which depends on the duration and intensity of the exercise, and the horizontal axis represents the time at which the excessive session was performed. Alternatively, the vertical axis of contextual window 206 could represent the estimated number of calories burned by the user during the exercise session and the horizontal axis represents the time at which the excessive session was performed. This contextual information can also be input by the user and stored on any of the aforementioned memory means, or in the local memory of the mobile communication device 108. Exercise information can also be retrieved from an automated activity tracker running on the mobile communication device 108 or another dedicated apparatus.

Contextual window 207 shows notes which can be input by a user. Such notes could include lifestyle, medical or any other type of information. Non-limiting examples of such notes could include "Forgot to take my meds this morning", or "Could not go for my run this afternoon (raining too hard)". For example, if a user selected the "notes" box and dragged it to a specific time in contextual information window 207, a popup window would allow the user to input a note associated with that specific time.

In some embodiments, navigation bar 211 provide information for use by a user. Such information could include, but is not limited to, contact information for the user's healthcare professional, account information, etc.

The description and drawings merely illustrate the principles of the invention. It will thus be appreciated that those skilled in the art will be able to devise various arrangements that, although not explicitly described or shown herein, embody the principles of the invention and are included within the scope of the appended claims.

For example, while trend window 201, magnified window 202 and various contextual information windows 202, 203, 204, 205, 206 and 207 have specific spatial relationships to each other in Figure 2, other spatial relationships may be envisaged without departing from the claimed invention. For example, trend window 201 could be position below the other windows or, alternatively, all windows could be laid out vertically from left to right or from right to left.

The functions of the various elements shown in the figures, may be provided through the use of dedicated hardware as well as hardware capable of executing software in association with appropriate software. When provided by a processor, the functions may be provided by a single dedicated processor, by a single shared processor, or by a plurality of individual processors, some of which may be shared.

Moreover, explicit use of the term "processor" should not be construed to refer exclusively to hardware capable of executing software, and may implicitly include, without limitation, digital signal processor (DSP) hardware, network processor, application specific integrated circuit (ASIC), field programmable gate array (FPGA), read only memory (ROM) for storing software, random access memory (RAM), and non volatile storage. Other hardware, conventional and/or custom, may also be included.

## Claims

1. A graphical user interface (200) for displaying blood glucose information, the graphical user interface (200) comprising:
a trend window (201) arranged to display blood glucose levels of a user on a timeline;
a magnifying window (210) superimposed on a segment of the timeline, the relative position of the magnifying window (210) with respect to the timeline being movable;
a magnified window (202) arranged to display, over an area which is larger than the magnifying window (210), the information contained in the segment of the timeline; and
at least one contextual information window (203, 204, 205, 206, 207) arranged to display, over an area of substantially the same size as the magnified window (202), information which is contextual to the blood glucose levels.

2. The graphical user interface (200) of claim 1, further comprising:
resizing means arranged to allow a user to resize the magnifying window (210) with respect to the trend window (201), wherein the resizing of the magnifying window (210) with respect to the trend window (201) changes the size of the segment of the timeline.

3. The graphical user interface (200) of any of the preceding claims arranged such that a user can reposition the timeline within the trend window (201).

4. The graphical user interface (200) of any of the preceding claims, further comprising:
magnifying window moving means arranged to move the magnifying window (210) with respect to the trend window (201), the relative position of the magnifying window (210) with respect to the timeline being moved by the magnifying window moving means.

5. The graphical user interface (200) of any of the preceding claims, comprising:
a plurality of contextual information windows (203, 204, 205, 206, 207) arranged to display, over an area of the same size as the magnified window (202), information which is contextual to the blood glucose levels, the plurality of contextual information windows (203, 204, 205, 206, 207) being arranged contiguously and in a particular order on the graphical user interface.

6. The graphical user interface (200) of claim 5, wherein:
each of the plurality of contextual information windows is arranged to be moveable, such that the particular order in which the contextual information windows (203, 204, 205, 206, 207) are arranged can be changed by a user.

7. The graphical user interface (200) of any of the preceding claims, further comprising:
at least one contextual information window (203, 204, 205, 206, 207) including a graphical input icon which is arranged to be dragged and dropped by a user onto a location of the at least one contextual information window (203, 204, 205, 206, 207), thereby creating an added reading to the at least one contextual information window (203, 204, 205, 206, 207), the location of the at least one contextual information window (203, 204, 205, 206, 207) being indicative of quantum and time of the added reading being input by the user into the at least one contextual information window (203, 204, 205, 206, 207).

8. The graphical user interface (200) of claim 7, wherein dragging and dropping of the graphical input icon onto the location of the at least one contextual information window (203, 204, 205, 206, 207) causes a popup window to appear, the popup window being arranged to allow a user to input further information in respect of the added reading and/or to adjust the location of the added reading in the at least one contextual information window (203, 204, 205, 206, 207).

9. The graphical user interface (200) of any of the preceding claims wherein the at least one contextual information window (203, 204, 205, 206, 207) is arranged to display quantitative and qualitative information on a timeline, the qualitative and quantitative information relating to one of: insulin injected by a user, insulin administered to a patient by way of an insulin pump, food consumed by a user and exercise sessions performed by a user.

10. An apparatus comprising:
processing means; and
a display device, the processing means being arranged to cause the display device to display a graphical user interface in accordance with any of claims 1 to 9.

11. A method of providing a graphical user interface (200) for displaying blood glucose information, the method comprising:
providing a trend window (201) arranged to display blood glucose levels of a user on a timeline;
providing a magnifying window (210) superimposed on a segment of the timeline, the relative position of the magnifying window (210) with respect to the timeline being movable;
providing a magnified window (202) arranged to display, over an area which is larger than the magnifying window (210), the information contained in the segment of the timeline; and
providing at least one contextual information window (203, 204, 205, 206, 207) arranged to display, over an area of the same size as the magnified window (202), information which is contextual to the blood glucose levels.

12. A computer program product for a data-processing device, the computer program product comprising a set of instructions which, when loaded into the data-processing device, causes the device to perform the steps of the method as claimed in claim 11.
